# EUROPÄISCHE PATENTSCHRIFT

(11) **EP 2 909 300 B1**
(45) Veröffentlichungstag und Bekanntmachung des Hinweises auf die Patenterteilung: **02.03.2016**
(21) Anmeldenummer: 13752859.2
(22) Anmeldetag: 02.08.2013
(51) Int. Cl.: C12M 1/00, C12M 1/06

(54) **BIOREAKTOR, REAKTORBEUTEL DAFÜR UND RÜHRER ZUR UMWÄLZUNG SEINES INHALTES**
BIOREACTOR, REACTOR POCKET FOR SAME AND STIRRER FOR CIRCULATING CONTENTS OF SAID POCKET
BIORÉACTEUR, SON ENVELOPPE DE RÉACTEUR, ET DISPOSITIF D'AGITATION PERMETTANT DE RECIRCULER SON CONTENU

(30) Priorität: 18.10.2012 DE 102012020384
(43) Veröffentlichungstag der Anmeldung: 26.08.2015
(73) Patentinhaber: Sartorius Stedim Biotech GmbH, 37079 Göttingen (DE)
(72) Erfinder: ZEUCH, Stefan, 37079 Göttingen (DE); REGEN, Thomas, 37079 Göttingen (DE); GRELLER, Gerhard, 37085 Göttingen (DE); REIF, Oscar-Werner, 30173 Hannover (DE)
(74) Vertreter: Schneider, Peter Christian
(86) Internationale Anmeldenummer: PCT/EP2013/066301
(87) Internationale Veröffentlichungsnummer: WO 2014/060130

(56) Entgegenhaltungen:
- EP-A1- 2 065 085
- WO-A1-2008/040567
- WO-A1-2009/116002
- DE-U1-202007 005 868
- DE-U1-202009 006 840
- US-A1- 2010 197 003

## Beschreibung

### Gebiet der Erfindung

Die Erfindung betrifft einen Bioreaktor, umfassend
- einen Reaktorbeutel mit einer überwiegend flexiblen Beutelwand, die zur Lagerung eines einer Umwälzung eines Beutelinhaltes dienenden Rührers einen steif ausgebildeten Lagerbereich aufweist,
- einen im Beutelinneren an dem Lagerbereich gelagerten, wellenlosen, bereichsweise permanentmagnetischen Rührer, und
- eine außerhalb des Reaktorbeutels positionierte Spulenanordnung, mit der ein rotierendes Magnetfeld erzeugbar ist, welches in ein Drehmoment auf den Rührer ausübender Weise mit dessen permanentmagnetischen Bereichen wechselwirkt.

Die Erfindung betrifft weiter einen Reaktorbeutel für einen Bioreaktor, umfassend eine überwiegend flexible Beutelwand, die zur Lagerung eines einer Umwälzung eines Beutelinhaltes dienenden, wellenlosen, wenigstens bereichsweise permanentmagnetischen Rührers einen steif ausgebildeten Lagerbereich aufweist.

Die Erfindung betrifft schließlich einen Rührer zur Umwälzung eines Inhaltes eines Bioreaktors, bereichsweise permanentmagnetisch ausgebildet und umfassend eine Mehrzahl flügelartiger, an einer Halterung fixierter Umwälzorgane.

### Stand der Technik

Derartige Bioreaktoren, Reaktorbeutel und Rührer dafür sind bekannt aus der US 8, 123, 199 B2.

Bekanntermaßen dienen Bioreaktoren als Behältnisse für Flüssigkeiten, in denen sich biologische Prozesse, wie beispielsweise Fermentation oder Zellwachstum in kontrollierter Weise abspielen sollen. Aufgrund der Stoffwechselaktivität der beteiligten Mikroorganismen kommt es zu lokalen Konzentrationsänderungen verschiedenster chemischer Komponenten. Um im gesamten Behältnis gleiche oder zumindest kontrollierte Konzentrationsbedingungen zu gewährleisten, ist es erforderlich, die Flüssigkeit im Reaktor gelegentlich, vorzugsweise ständig umzurühren. Zur Vermeidung von Kontaminationen sind die Rührer in der Regel im Inneren des ansonsten (bis auf definierte Zu- und Ableitungen) geschlossenen Reaktorbehältnisses angeordnet. Eine Schwierigkeit besteht darin, die im Inneren des Reaktorbehältnisses befindlichen Rührer durch in der Regel außerhalb des Reaktorbehältnisses angeordnete Antriebsvorrichtungen in die gewünschte Rührbewegung zu versetzen.

Bei Bioreaktoren mit starren Wandungen, beispielsweise Stahltanks, ist es bekannt, die eigentlichen Umwälzorgane des Rührers, beispielsweise Rührflügel, an einer Welle zu fixieren, die die Behälterwandung in einem abgedichteten Lager durchsetzt, sodass die Rührerwelle in einfacher Weise mit der Antriebswelle eines beliebig gestalteten Motors gekoppelt werden kann.

In jüngster Zeit nimmt der Einsatz von Bioreaktoren, deren eigentliches Behältnis als flexibler Beutel gestaltet und zum Einmalgebrauch eingerichtet ist, stetig zu. Vorteile liegen in der kostengünstigen Herstellung der Folienbeutel, der einfachen und platzsparenden Lagerung, der leichten Sterilisierbarkeit und Kontaminationssicherheit und dem Entfall einer aufwendigen Reinigung nach Gebrauch. Allerdings ist das Problem des angemessenen Umrührens des Beutelinhaltes noch nicht abschließend gelöst. Insbesondere werden Varianten mit die Beutelwand durchsetzenden Rührerwellen als nachteilig angesehen, da dies die Kontaminationsgefahr erhöht, den Platzbedarf für die Lagerung deutlich steigert und die Herstellung des Beutels durch die Notwendigkeit eines abgedichteten Wellendurchlasses verkompliziert und verteuert.

Aus der gattungsbildenden US 8,123,199 B2 ist ein wellenloser Rührer bekannt, der an einer flach zylindrischen Halterung eine Mehrzahl von Rührflügeln aufweist, wobei die flach zylindrische Halterung in einem korrespondierend geformten, steifen Topf, der den Boden des Reaktorbeutels bildet, gelagert ist. Die zylindrische Halterung ist wenigstens bereichsweise permanentmagnetisch ausgebildet und wechselwirkt mit einem äußeren, die Topfwandung durchsetzenden magnetischen Feld. Das äußere Magnetfeld ist als Drehfeld gestaltet, sodass die Rührerhalterung zusammen mit den flügelartigen Umwälzorganen in ihrem Lagertopf in Rotationsbewegung versetzt wird. Diese bekannte Vorrichtung weist mehrere Nachteile auf. Zum einen ist sie auf die Anordnung des Rührers im Bodenbereich des Reaktorbeutels beschränkt, sodass bei hohen Bioreaktoren mit einer ungenügenden Durchmischung des Reaktorinhaltes zu rechnen ist. Zum anderen ist der Rührer in seinem Lagertopf nicht axial fixiert - im Gegenteil wird zur Reduktion der Lagerreibung ein axiales Anheben des Rührers durch das äußere Magnetfeld beschrieben. Dies führt dazu, dass der Rührer beim Transport leicht aus seiner Lagerung fallen und mit seinen scharfkantigen Flügeln die Beutelwandung beschädigen kann.

### Aufgabenstellung

Es ist die Aufgabe der vorliegenden Erfindung, gattungsgemäße Bioreaktoren, Reaktorbeutel und Rührer derart weiterzubilden, dass die Nachteile des Standes der Technik überwunden werden. Insbesondere sollen Bioreaktoren bzw. Reaktorbeutel bereitgestellt werden, die mit einem deutlich verringerten Stauraumbedarf lagerfähig bzw. transportierbar sind. Weiterhin sollen eine verbesserte Durchmischung des Reaktorinhaltes und sicherere Transport- und Lagermöglichkeiten erzielt werden.

### Darlegung der Erfindung

Diese Aufgabe wird in Verbindung mit den Merkmalen des Oberbegriffs von Anspruch 1 dadurch gelöst, dass in einem vorzugsweise nicht randständigen Höhenabschnitt des Reaktorbeutels als Lagerbereich ein steifer Profilring aus einem unmagnetischen Werkstoff, z. B. Kunststoff, angeordnet ist, an dem der als den Beutelquerschnitt überspannender Rotor ausgebildete Rührer mittels einer Schienenführung drehbeweglich gelagert ist,wobei das Magnetfeld der elektrischen Spulenanordnung mit wenigstens einem im radial äußeren Bereich des Rotors angeordneten Permanentmagneten wechselwirkt.

Die Aufgabe wird weiter in Verbindung mit den Merkmalen des Oberbegriffs von Anspruch 11 dadurch gelöst, dass in einem vorzugsweise nicht randständigen Höhenabschnitt des Reaktorbeutels als Lagerbereich ein steifer Profilring aus einem unmagnetischen Werkstoff, z. B. Kunststoff, angeordnet ist, an dem ein als den Beutelquerschnitt überspannender Rotor ausgebildeter Rührer mittels einer Schienenführung drehbeweglich axial und radial lagerbar ist.

Die Aufgabe wird schließlich in Verbindung mit den Merkmalen des Oberbegriffs von Anspruch 14 dadurch gelöst, dass der Rührer als ein einen Zentralkörper und wenigstens drei sich von dem Zentralkörper radial erstreckende Speichen aufweisender Rotor ausgebildet ist, der in seinem radial äußeren Bereich mit wenigstens einem Permanentmagneten versehen ist und wenigstens ein Führungsglied einer äußeren Schienenführung aufweist.

Bevorzugte Ausführungsformen der Erfindung sind Gegenstand der abhängigen Patentansprüche.

Wesentlicher Gegenstand der Erfindung ist die spezielle Ausgestaltung des Lagerbereichs und die damit in Zusammenhang stehende korrespondierende Ausgestaltung des Rührers. Der Rührer überspannt den gesamten Beutelquerschnitt und ist in seinem radialen Außenbereich an einem die Schiene einer Schienenführung bildenden Profilring, welcher den Umfang des Reaktorbeutels umläuft, gelagert. In diesem radial außen gelegenen, mechanischen Wechselwirkungsbereich zwischen dem Rotor und seiner Führung findet auch die magnetische Wechselwirkung zum motorischen Antrieb des Rührers statt. Die magnetischen Kräfte, die zwischen der äußeren Spulenanordnung und dem oder den im Außenbereich des Rührers angeordneten Permanentmagneten wirken, durchsetzen den Profilring, sodass dieser aus einem nicht-magnetischen Werkstoff, z. B. Kunststoff, ausgebildet zu sein hat. Die gesamte Rühreranordnung kann daher so gestaltet sein, dass sie nur sehr geringen axialen Bauraum beansprucht und überdies an jeder beliebigen Axialposition des Reaktionsbeutels angeordnet sein kann. Auch ist es denkbar, mehrere derartige Rühreranordnungen an unterschiedlichen Axialpositionen des Reaktorbeutels anzuordnen, sodass auch bei sehr hohen Bioreaktoren eine ausreichende Durchmischung des gesamten Reaktorinhaltes gewährleistet werden kann. Aufgrund der geringen axialen Bauhöhe jeder Rühreranordnung werden die an sich flexiblen Wände des Reaktorbeutels nur rippenartig versteift, wobei zwischen den diese "Rippen" bildenden Profilringen flexible Wandungsbereiche verbleiben, sodass der Reaktorbeutel zur Lagerung und zum Transport faltenbalgartig zusammengeschoben werden kann, wodurch der benötigte Stauraum deutlich reduziert wird. Dabei kann bei geeigneter Ausgestaltung der Schienenführung auch kein Rotor aus seiner Führung springen, sodass im zusammengelegten Zustand keine Beschädigung der Beutelwand zu befürchten ist und im entfalteten Zustand ohne vorangehende Montageschritte unverzüglich der Rührbetrieb aufgenommen werden kann.

Bei einer bevorzugten Ausführungsform durchsetzt der Profilring die flexible Beutelwand und ist stoffschlüssig mit ihr verbunden. Durch die stoffschlüssige Verbindung, die beispielsweise durch Schweißen oder Kleben erfolgen kann, wird die Dichtigkeit des Reaktorbeutels auch im Bereich der Rühreranordnung gewährleistet. Dadurch, dass der Profilring die Beutelwand radial durchsetzt, wird die direkte Zugänglichkeit seiner Außenseite von außerhalb des Reaktorbeutels gewährleistet. Diesem Merkmal kommt im Zusammenhang mit weiter unten beschriebenen, speziellen Ausgestaltungen des motorischen Rührerantriebs besondere Bedeutung zu. Allerdings ist grundsätzlich auch denkbar, dass der Profilring stoffschlüssig an der Innenseite der ansonsten flexiblen Beutelwandung festgelegt ist, wobei die Beutelwandung die radial äußeren Konturen des Profilrings dicht anliegend nachzeichnet. Diese Variante wird jedoch in der Regel Vorrichtungen mit sehr dünnem, vorzugsweise elastischem Beutelwandungsmaterial vorbehalten sein.

Bevorzugt weist der Profilring eine umlaufende, zum Beutelinneren hin offene Führungsnut auf, in der wenigstens ein radial außen an dem Rotor angeordneter Führungsschuh axial und radial geführt ist. Auf spezielle Ausgestaltungsvarianten des Führungsschuhs bei verschieden ausgebildeten Rotoren soll weiter unten eingegangen werden. Bei allen derartigen Ausführungsformen wird jedoch der Führungsschuh wenigstens dreiseitig von der Führungsnut umgriffen, sodass sowohl eine radiale als auch eine axiale Lagerung gewährleistet ist.

Alternativ ist auch denkbar, dass der Profilring einen umlaufenden, zum Beutelinneren hin gerichteten Grad aufweist, der in wenigstens ein radial außen an dem Rotor angeordnetes Nutelement hineinragt, sodass der Rotor axial und radial geführt ist. Diese Variante stellt im Wesentlichen die kinematische Umkehr der zuvor beschriebenen, bevorzugten Ausführungsform dar. Für den Fachmann wird es ein leichtes sein, die weiter unten detailliert beschriebenen Ausführungsformen des nutgeführten Führungsschuhs im Sinne dieser kinematischen Umkehr auf entsprechende Ausführungsformen mit gradgeführtem Nutelement zu übertragen.

Zur Reduzierung der Reibung ist bei einer Weiterbildung der Erfindung vorgesehen, dass an wenigstens drei Umfangspositionen des Profilrings radial ausgerichtete Wälzlagerrollen und/oder axial ausgerichtete, gegenständige Paare von Wälzlagerrollen federnd in die Führungsnut hineinragen bzw. bei Ausführungsformen mit Führungsgrad aus diesem herausragen.

Alternativ oder zusätzlich hierzu kann vorgesehen sein, dass an wenigstens drei Umfangspositionen des Profilrings radial ausgerichtete Gleitlagerfedern und/oder axial ausgerichtete, gegenständige Paare von Gleitlagerfedern in die Führungsnut hineinragen bzw. bei Ausführungsformen mit Führungsgrad, aus diesem herausragen. Die Oberflächen der Gleitlagerfedern sind dabei bevorzugt mit reibungsminderndem Material, beispielsweise Polytetrafluorethylen beschichtet. Grundsätzlich ist es selbstverständlich auch denkbar, derartige oder ähnliche Reibungsminderungselemente am Rotor, insbesondere am Führungsschuh bzw. am Nutelement vorzusehen. Der Grund für die allgemeine Bevorzugung von Ausführungsformen mit Führungsnut gegenüber Ausführungsformen mit Führungsgrad, liegt in deren spezieller Eignung für eine besonders vorteilhafte Weiterbildung der Erfindung. Demnach ist vorgesehen, dass die Führungsnut auf der Außenseite des Profilrings einen Vorsprung mit wenigstens einer Axialanlagefläche bildet, an der eine als separate Einheit oder Einheitengruppe ausgebildete elektrische Spulenanordnung angelegt bzw. anlegbar ist, mit der das magnetische Feld erzeugbar ist. Unter einer Axialanlagefläche ist in diesem Zusammenhang eine Fläche mit parallel zur Axialrichtung ausgerichteter Flächennormale zu verstehen, an welche ein Anlageelement, hier insbesondere die elektrische Spulenanordnung, so anlegbar ist, dass eine radiale Überlappung zustande kommt. Hierdurch kann nämlich eine radiale Überlappung des im Inneren der Führungsnut geführten Führungsschuhs mit der an der äußeren Axialanlagefläche angelegten Spulenanordnung realisiert werden, was zu einer Minimierung der Distanz zwischen der das Magnetfeld erzeugenden Spulenanordnung und einem im Außenbereich des Rührers, ggf. sogar im Bereich des Führungsschuhs angeordneten Permanentmagneten führt. Hierdurch kann die Wechselwirkung zwischen dem Permanentmagneten und der Spulenanordnung maximiert werden, sodass zur Erzielung einer vorgegebenen Antriebskraft nur kleine Permanentmagneten und/oder geringe Spulenströme erforderlich sind. Günstigerweise liegt nicht nur eine Axialanlagefläche vor. Vielmehr wird bevorzugt, dass die Führungsnut auf der Außenseite des Profilrings dreiseitig von einer als separate Einheit oder Einheitengruppe ausgebildeten elektrischen Spulenanordnung, mit der das magnetische Feld erzeugbar ist, formschlüssig umgriffen ist. Durch diese Maßnahme kann die Wechselwirkung zwischen Spulen und Permanentmagneten nochmals gesteigert werden.

Der Fachmann erkennt, dass die rückwärtige Zugänglichkeit des Profilrings im Zusammenhang mit dieser Ausführungsform von besonderer Bedeutung ist. Insbesondere wird dadurch eine günstige Grenze zwischen zum Einmalgebrauch vorgesehenen Elementen (Beutel, Rotor und Profilring) und wiederverwendbaren Elementen (Spuleneinsätze) gezogen.

Bzgl. der Gestaltung des Rotors ist bevorzugt vorgesehen, dass dieser einen Zentralkörper und wenigstens drei sich radial von dem Zentralkörper erstreckende Speichen aufweist. Dies kann beispielsweise durch eine propellerartige Gestaltung des Rotors realisiert werden, wobei die Speichen als hydrodynamisch günstig angestellte Flügel gestaltet sind, um die gewünschte Durchmischung des Reaktorinhaltes zu bewerkstelligen und um auftretende Scherkräfte, die auf Zellen in Zellkulturen innerhalb des Bioreaktors einwirken, zu minimieren.

Bei einer anderen bevorzugten Ausgestaltung ist vorgesehen, dass die Speichen des Rotors durch eine ringförmige Felge miteinander verbunden sind. Der Rotor hat bei dieser Ausführungsform die Gestalt eines Speichenrades, wobei die eigentlichen Umwälzorgane, d.h. die Rührflügel auch bei dieser Ausgestaltung durch die spezielle Formgebung der Speichen realisiert sein können.

Bei beiden genannten Ausführungsformen, d.h. Propeller- und Speichenradgestaltung des Rotors, können die Umwälzorgane auch im Bereich des Zentralkörpers angeordnet sein. In diesem Fall wären die Speichen möglichst so zu gestalten, dass sie der Rotation des Rotors nur einen möglichst geringen hydrodynamischen Widerstand entgegensetzen.

Während bei der Propellergestaltung des Rotors die mit der Spulenanordnung wechselwirkenden Permanentmagneten zusammen mit den als Kulissensteinen oder Nutelementen ausgestalteten Führungsgliedern im Bereich der Propellerflügelspitzen angeordnet sind, ist bei der Speichenradgestaltung des Rotors bevorzugt vorgesehen, dass die Felge mit einer Mehrzahl über ihren Umfang verteilter Permanentmagnete versehen ist. Je nach Ausführungsform kann die Felge selbst als ringförmiger Führungsschuh dienen. Alternativ können mehrere isolierte Bereiche der Felge als kulissensteinartige Führungsschuhe ausgestaltet sein, während der dazwischen liegende Felgenbereich lediglich als Magnetträger dient. Auch ist denkbar, dass die Speichen die Felge radial durchstoßen, sodass sich eine schiffsteuerradartige Struktur ergibt, wobei die Magnete auf dem Felgenring und kulissensteinartige Führungsschuhe oder Nutelemente im Spitzenbereich der Speichen liegen. Auch die umgekehrte Anordnung ist denkbar, bei der die Magnete im Spitzenbereich der Speichen liegen, während den dazwischen liegenden Felgenbögen die Aufgabe der Führungsglieder zukommt. Diese Variante erschwert allerdings die Radialführung.

Der Fachmann wird erkennen, dass der motorische Antrieb vorzugsweise nach Art eines Linearmotors mit kreisförmig gebogenem Langstator ausgebildet ist, bei dem die die Magnete oder Magnetgruppen tragenden Abschnitte des Rotors als miteinander verbundene Läufer angesehen werden können. Durch Ansteuerung der Spulenanordnungen zur Erzeugung eines rotierenden Magnetfeldes, beispielsweise in Form eines dreiphasigen Drehstroms werden die Läufer synchron zueinander auf der durch die Schienenführung des Profilrings vorgegebenen Kreisbahn bewegt. Durch die mechanische Verbindung der Läufer in dem propeller- oder speichenradförmigen Rotor entsteht eine Drehbewegung des letzteren. Eine solche Drehbewegung ist selbstverständlich auch erzielbar, wenn nur ein einziger Läufer, d.h. nur ein einziger einen oder mehrere Magneten tragender Rotorbereich vorgesehen ist.

Weitere Merkmale und Vorteile der Erfindung ergeben sich aus der nachfolgenden speziellen Beschreibung und den Zeichnungen.

### Kurzbeschreibung der Zeichnungen

Es zeigen:
- Figur 1:: eine schematisierte Querschnittsdarstellung eines erfindungsgemäßen Bioreaktors,
- Figur 2:: eine perspektivische Schnittdarstellung des erfindungsgemäßen Bioreaktors,
- Figur 3:: eine Ausschnittsvergrößerung aus Figur 1,
- Figur 4:: eine perspektivische Darstellung eines erfindungsgemäßen Rührers,
- Figur 5:: eine Draufsicht auf den Rührer von Figur 4,
- Figur 6:: eine Seitenansicht des Rührers von Figur 4,
- Figur 7:: eine perspektivische Darstellung eines Segmentes eines Profilrings zur Rührerlagerung,
- Figur 8:: eine Seitenansicht der Innenseite des Profilringsegmentes von Figur 7,
- Figur 9:: eine Schnittdarstellung des Profilringsegmentes von Figur 7 entlang der Schnittlinie X-X in Figur 8,
- Figur 10:: eine Schnittdarstellung des Profilringsegmentes von Figur 7 entlang der Schnittlinie IX-IX in Figur 8,
- Figur 11:: eine perspektivische Darstellung eines Spuleneinsatzes,
- Figur 12:: eine Draufsicht auf den Spuleneinsatz von Figur 11,
- Figur 13:: eine Seitenansicht der Außenseite des Spuleneinsatzes von Figur 11,
- Figur 14:: eine perspektivische Darstellung einer aus Rührer, Profilring und Spuleneinsätzen zusammengesetzten Rühreranordnung,
- Figur 15:: eine schematische Darstellung einer möglichen Spulen/Magnetkonstellation,
- Figur 16:: eine schematische Darstellung einer alternativen Spulen/Magnetkonstellation.

### Ausführliche Beschreibung bevorzugter Ausführungsformen

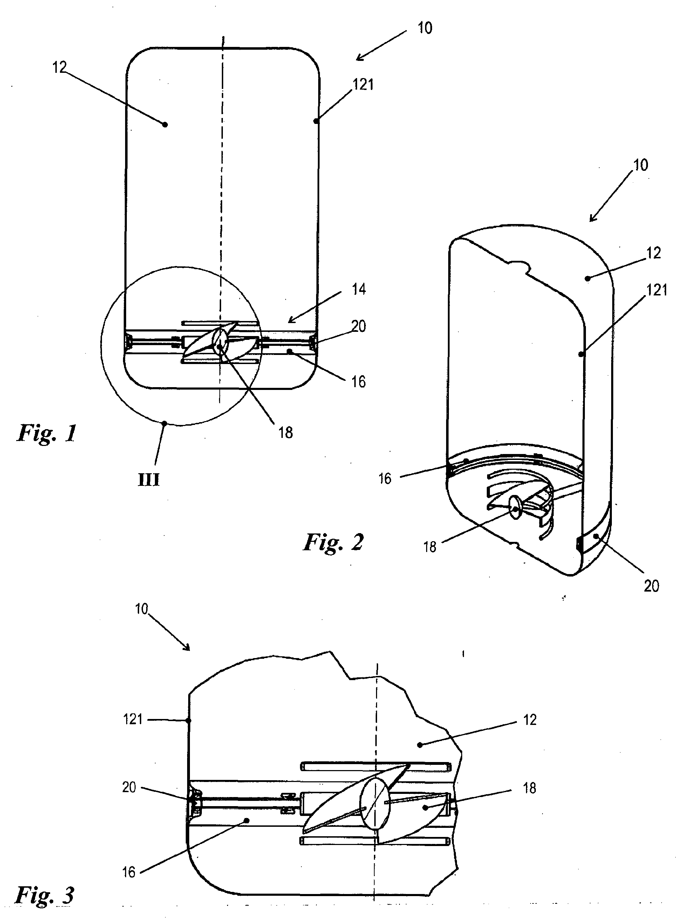

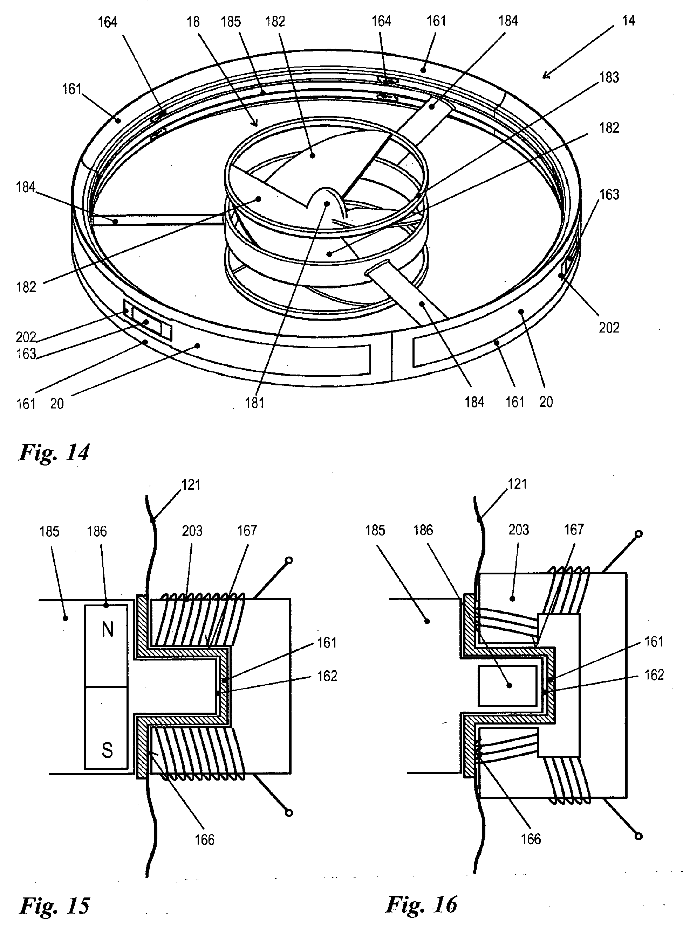

Die Figuren 1 bis 14 illustrieren beispielhaft eine bevorzugte Ausführungsform der Erfindung und sollen, soweit nicht speziell Bezug auf eine bestimmte Figur genommen wird, gemeinsam diskutiert werden. Die Figuren 15 und 16 stellen rein schematisch und beispielshaft zwei mögliche Spulen/Magnet-Konstellationen zur Realisierung des motorischen Antriebs einer erfindungsgemäßen Rühreranordnung, insbesondere einer Rühreranordnung gem. den Figuren 1 bis 14 dar. Gleiche Bezugszeichen in den Figuren deuten auf gleiche oder analoge Elemente hin.

In den Figuren 1 bis 3 ist in stark schematisierter Darstellung ein Bioreaktor 10 gezeigt, der einen Reaktorbeutel 12 und eine Rühreranordnung 14 aufweist. Der Reaktorbeutel 12 hat eine im Wesentlichen flexible Beutelwandung 121, die lediglich im Bereich der Rühreranordnung 14 durch deren Profilring 16 aus einem steifen, unmagnetischen Werkstoff, z.B. Kunststoff, versteift ist. Bevorzugt ist die Beutelwandung 121 im Bereich der Rühreranordnung 14 unterbrochen und wenigstens bereichsweise durch den Profilring 16 ersetzt. Dieser kann beispielsweise in seinen axialen Randbereichen mit der Beutelwandung verklebt oder verschweißt sein. Dabei ist es irrelevant, ob die stoffschlüssige Verbindung des Profilrings 16 mit der Innen- oder der Außenseite der Beutelwandung 121 erfolgt. Im Ergebnis bilden die Beutelwandung 121 und der Profilring 16 gemeinsam einen abgedichteten Behälter, der lediglich im Bereich des Profilrings rippenartig versteift und ansonsten flexibel ist. Die Außenseite des Profilrings 16 ist dabei vom Beuteläußeren her zugänglich.

In den Figuren 1 bis 3 nicht dargestellt sind üblicherweise bei Bioreaktoren vorgesehene Zu- und Ableitungen, die vom Fachmann je nach Anforderungen des konkreten Einzelfalls ergänzt werden können. Ebenfalls nicht dargestellt ist ein Beutelhalter, der als Träger- bzw. Stützvorrichtung zum Aufhängen bzw. Stützen des Reaktorbeutels 12, als Trägerplatte zum Auflegen des Reaktorbeutels 12 oder als exoskelettartiges Behältnis zur Aufnahme des Reaktorbeutels 12 ausgebildet sein kann. Insbesondere in letzterem Fall ist es möglich, die weiter unten noch näher beschriebenen Spuleneinsätze in der Wandung des Exoskelett-Behälters anzuordnen, sodass sie beim Einsetzen des Reaktorbeutels 12 bzw. beim Schließen des Exoskelett-Behälters automatisch in ihre Einsatzpositionen im Profilring eingeführt werden.

Neben dem Profilring 16 weist die Rühreranordnung einen drehbeweglich angeordneten und im Profilring axial und radial gelagerten Rotor 18 sowie die bereits vorerwähnten, in den Figuren 1 bis 3 jedoch kaum erkennbaren Spuleneinsätze 20 auf. Eine vom Reaktorbeutel 12 isolierte Rühreranordnung 14 ist in Figur 14 dargestellt. Ihre einzelnen Elemente sollen nachfolgend im Zusammenhang mit den übrigen Figuren detaillierter beschrieben werden.

Der Fachmann wird unschwer erkennen, dass der erfindungsgemäße Bioreaktor 10 nicht auf Varianten mit nur einer Rühreranordnung 14 beschränkt ist. Vielmehr ist es möglich, insbesondere bei sehr hohen Bioreaktoranordnungen, mehrere Rühreranordnungen 14 in unterschiedlichen axialen Höhen zu positionieren. Aufgrund der Flexibilität der Beutelwandung 121 im Bereich zwischen den Rühreranordnungen 14 ergibt sich eine faltenbalg- oder ziehharmonikaartige Faltbarkeit, die auch bei mehreren Rühreranordnungen 14 zu einem sehr geringen Stauraum führt.

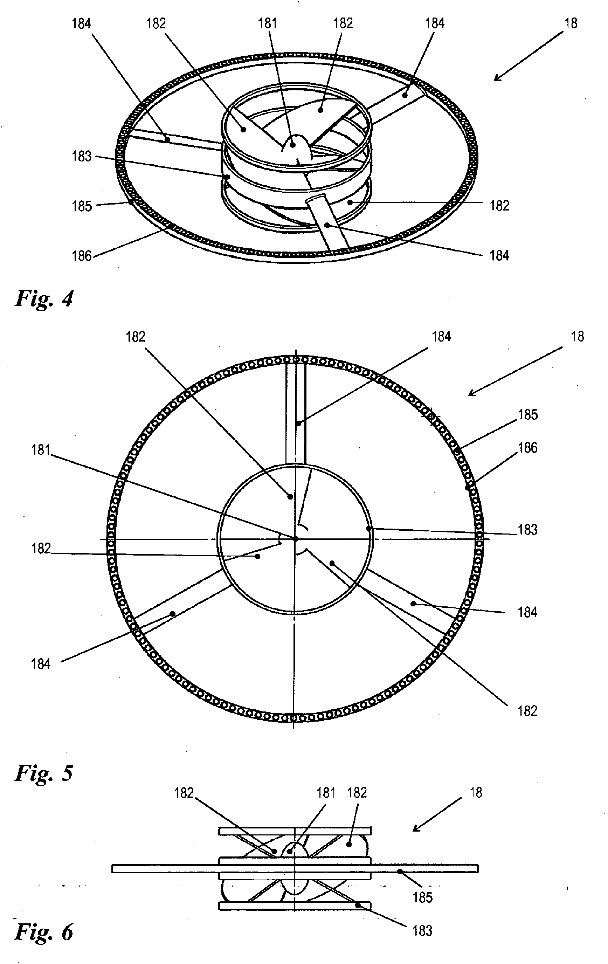

Figuren 4 bis 6 zeigen in unterschiedlichen Darstellungen eine bevorzugte Ausführungsform eines Rotors 18. Die eigentliche Rührfunktion wird durch die Elemente im Zentralbereich des Rotors 18 erfüllt. Es sind dies bei der dargestellten Ausführungsform ein Zentralkörper 181, der bevorzugt, wie dargestellt, in einer strömungsgünstigen Form gestaltet ist, welche einer Axialströmung besonders geringen hydrodynamischen Widerstand entgegensetzt. An den Zentralkörper 181 angeformt sind schiffschraubenartige Flügel 182, welche als eigentliche Umwälzelemente anzusehen sind. Die spezielle Gestaltung der Rührflügel 182 kann nach üblichen hydrodynamischen Kriterien in Ansehung der Erfordernisse des Einzelfalls, insbesondere unter Berücksichtigung der Viskosität der umzurührenden Flüssigkeit sowie der gewünschten Drehzahl, vorgenommen werden. Die aus Zentralkörper 181 und Rührflügeln 182 bestehende Rührschraube ist bei der dargestellten Ausführungsform in einem Käfig 183 angeordnet, der einerseits der Stabilisierung der Rührschraube und andererseits ihrer mechanischen Verbindung zu dem weiter unten beschriebenen motorischen Antrieb dient. Hierzu sind mehrere Speichen 184, die sich radial erstrecken, an dem Käfig 183 angeformt. Bevorzugt weisen diese Speichen 184 ein strömungsgünstiges Profil auf. Was im Detail dabei als günstig anzusehen ist, hängt von den Anforderungen des Einzelfalls ab. So kann eine möglichst widerstandsarme Profilform gewählt werden; andererseits ist es auch möglich, die Speichen 184 ähnlich den Rührflügeln 182 als wirksame Umwälzorgane zu gestalten. Die Speichen 184 verbinden den Käfig 183 mit einem Felgenring 185, der, wie nachfolgend detaillierter beschrieben, drehbeweglich in dem Profilring 16 gelagert ist. Der Felgenring 185 dient somit als den gesamten Umfang umlaufender Führungsschuh. Der Felgenring 185 hat bei der gezeigten Ausführungsform zudem die Aufgabe als Träger einer Vielzahl von Permanentmagneten 186, die in ihn eingelassen sind. Dies erfolgt vorzugsweise so, dass die Magnete 186 axial nicht über den Felgenring überstehen. Bevorzugt sind sie in das Kunststoffmaterial des Felgenrings 185 eingegossen.

Bei einer alternativen, nicht dargestellten Ausführungsform kann auf einen durchgehenden Felgenring 185 verzichtet werden. Bei dieser Ausführungsform sind lediglich die radial äußeren Spitzen der Speichen 184 als isolierte Führungsschuhe ausgebildet, wobei einer oder mehrere davon die Magnete 186 tragen können.

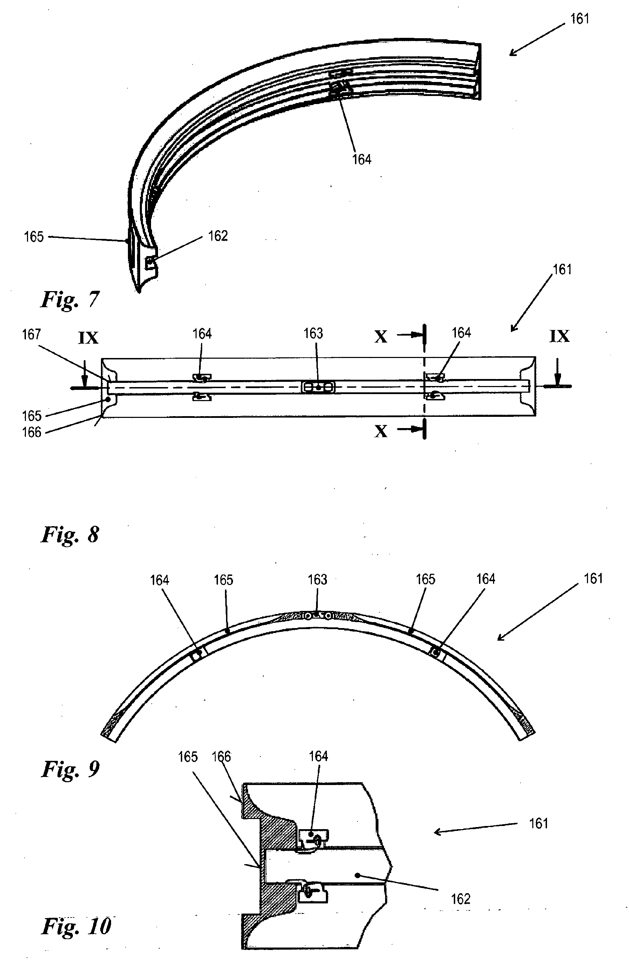

In den Figuren 7 bis 10 ist ein Segment 161 des Profilrings 16 in einer besonders bevorzugten Ausbildungsform dargestellt. Grundsätzlich ist es zwar möglich, die Grundstruktur des Profilrings 16 einstückig zu gestalten. Im Hinblick auf die Montage eines erfindungsgemäßen Reaktorbeutels bzw. eines erfindungsgemäßen Bioreaktors hat es sich jedoch als vorteilhaft erwiesen, den Profilring 16 in Form mehrerer, vorzugsweise identischer Profilringsegmente 161 zu gestalten.

Die Grundstruktur des Profilringsegmentes 161 besteht aus einem gekrümmten Kunststoffprofilstrang, der auf seiner Innenseite eine Führungsnut 162 aufweist. Diese Nut ist in ihrer Höhe und Tiefe auf den Felgenring 185 des Rotors 18 abgestimmt, sodass dieser in der Führungsnut 162 eine axiale und radiale Lagerung mit geringem Spiel erfährt. Die geeignete Größe des Spiels ist vorzugsweise auf den beabsichtigten Reaktorinhalt abzustimmen. Insbesondere bei Zellkulturen kann das Vorsehen eines zu kleinen Spiels zu unerwünschtem Zerquetschen von Zellen, die zwischen Felgenring 185 und Führungsnut 162 geraten, führen. Daher wird das Spiel bevorzugt größer als für die Erhaltung der Drehbeweglichkeit des Rotors 18 mindestens erforderlich gewählt und eine Nutation des Rotors 18 durch spezielle Wälz- oder Gleitlagerelemente 163, 164 verhindert. Bei der gezeigten Ausführungsform sind daher über den Umfang des Profilrings 16 verteilt mehrere Radiallager 163 und Axiallager 164 vorgesehen. Bei der bevorzugten Ausführungsform bestehen die Radiallager 163 jeweils aus zwei radial federgelagerten Rollen, deren Drehachse parallel zur Rotationsachse des Rotors 18 ausgerichtet ist. Diese federgelagerten Rollenpaare ragen von außen radial in die Führungsnut 162 hinein. Sind wenigstens (vorzugsweise genau) drei solcher Radiallager 163 vorgesehen, ist der Felgenring 185 des Rotors 18 elastisch in zentrierter Position gehalten. Die Axiallager 164 sind bei der dargestellten Ausführungsform als axial beidseitig der Führungsnut 162 einander gegenüberliegende federvorgespannte Rollenpaare mit radial ausgerichteten Drehachsen ausgestaltet, die den Felgenring 185 des Rotors 18 axial elastisch in der Führungsnut 162 zentrieren.

Insbesondere in den Figuren 7 und 10 ist deutlich erkennbar, dass das Profilringsegment 161 auf seiner Außenseite eine Einbuchtung 165 aufweist. Diese wird axial beidseitig von Radialanlageflächen 166 flankiert, die zur Fixierung der flexiblen Beutelwandung 121 am Profilring 16 dienen. Insbesondere können sie als Kontaktflächen für eine stoffschlüssige Verbindung, insbesondere eine Verklebung oder Verschweißung dienen. Im Bereich eines Lagers 163, 164 ist die Einbuchtung 165, wie insbesondere in Figur 9 und 10 erkennbar, weniger tief ausgebildet bzw. unterbrochen. Zwischen den Lagern hingegen ist die Einbuchtung 165 so tief, dass sich eine radiale Überlappung von Einbuchtung 165 und Führungsnut 162 ergibt. Mit anderen Worten bildet hier die radiale Außenwand der Führungsnut 162 eine Axialanlagefläche 167, an die die elektrischen Spulen angelegt werden können und dort in einem minimalen Abstand zu den Permanentmagneten 186 in dem in der Führungsnut 162 geführten Felgenring 185 positioniert sind.

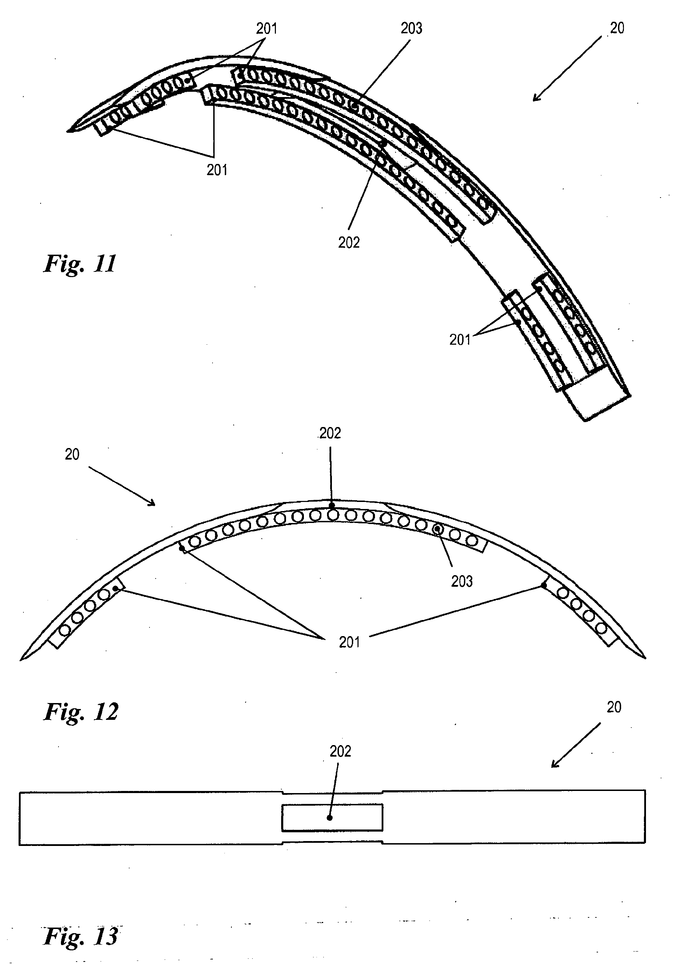

Die Figuren 11 bis 13 zeigen verschiedene Darstellungen einer Spulenanordnung 20, die korrespondierend zu der Einbuchtung 165 in der Außenseite der Profilringsegmente 161 geformt ist. Bei der dargestellten Ausführungsform weist die Spulenanordnung 20 an ihren axialen Kanten Spulenträgerstege 201 auf, die im zusammengesetzten Zustand der Rühreranordnung 14 an den Axialanlageflächen 167 der Profilringsegmente 161 zur Anlage kommen. Die Spulenträgerstege 201 sind bereichsweise unterbrochen, um Raum für die Axiallager 164 zu bieten. Im Bereich der Radiallager 163 weisen die Spuleneinsätze 20 Fenster 202 auf, die den für die Lagerelemente benötigten Raum zur Verfügung stellen.

In den Figuren sind die eigentlichen Spulen 203 nur schematisch angedeutet. Ihre exakte Gestaltung, Ausrichtung und elektrische Kontaktierung ist nicht gezeigt, kann jedoch vom Fachmann im Detail jeweils in Ansehung der Erfordernisse des Einzelfalls ausgeführt werden. Figuren 15 und 16 zeigen zwei mögliche Ausgestaltungen einer Spule in stark schematisierter Darstellung. Selbstverständlich wird der Fachmann die konkrete Anordnung der Permanentmagneten 186 in geeigneter Weise auf die spezielle Struktur der Spulen 203 abzustimmen haben. Auch die koordinierte Ansteuerung der Spulen oder Spulengruppen im Sinne eines kreisförmig gekrümmten Linearmotors kann der Fachmann unter bekannten elektrotechnischen Aspekten gestalten. Rein beispielhaft seien hier die üblichen Ausrichtungen der Permanentmagnete 186 in radialer Magnetisierung, axialer Magnetisierung und Halbach-Magnetisierung erwähnt, die jeweils entsprechende Spulenanordnungen und -ansteuerungen erfordern.

Natürlich stellen die in der speziellen Beschreibung diskutierten und in den Figuren gezeigten Ausführungsformen nur illustrative Ausführungsbeispiele der vorliegenden Erfindung dar. Dem Fachmann ist im Lichte der hiesigen Offenbarung ein breites Spektrum von Variationsmöglichkeiten an die Hand gegeben.

### Bezugszeichenliste

- 10: Bioreaktor
- 12: Reaktorbeutel
- 121: Beutelwandung
- 14: Rühreranordnung
- 16: Profilring
- 161: Profilringsegment
- 162: Führungsnut
- 163: Radiallager
- 164: Axiallager
- 165: Einbuchtung
- 166: Radialanlagefläche
- 167: Axialanlagefläche
- 18: Rotor/Rührer
- 181: Zentralkörper
- 182: Rührflügel
- 183: Käfig
- 184: Speiche
- 185: Felgenring
- 186: Permanentmagnet
- 20: Spuleneinsatz
- 201: Spulenträgersteg
- 202: Fenster
- 203: Spule

## Patentansprüche

1. Bioreaktor, umfassend
- einen Reaktorbeutel (12) mit einer überwiegend flexiblen Beutelwand (121), die zur Lagerung eines einer Umwälzung eines Beutelinhaltes dienenden Rührers (18) einen steif ausgebildeten Lagerbereich aufweist,
- einen im Beutelinneren an dem Lagerbereich gelagerten, wellenlosen, bereichsweise permanentmagnetischen Rührer (18), und
- eine außerhalb des Reaktorbeutels (12) positionierte Spulenanordnung (20), mit der ein rotierendes Magnetfeld erzeugbar ist, welches in ein Drehmoment auf den Rührer (18) ausübender Weise mit dessen permanentmagnetischen Bereichen wechselwirkt,
**dadurch gekennzeichnet,**
**dass** in einem Höhenabschnitt des Reaktorbeutels (12) als Lagerbereich ein steifer Profilring (16) aus einem unmagnetischen Werkstoff angeordnet ist, an dem der als den Beutelquerschnitt überspannender Rotor (18) ausgebildete Rührer mittels einer Schienenführung (162/185) drehbeweglich gelagert ist,
wobei das Magnetfeld der elektrischen Spulenanordnung (20) mit wenigstens einem im radial äußeren Bereich des Rotors (18) angeordneten Permanentmagneten (186) wechselwirkt.

2. Bioreaktor nach Anspruch 1,
**dadurch gekennzeichnet,**
**dass** der Profilring (16) die flexible Beutelwand (121) durchsetzt und mit ihr stoffschlüssig verbunden ist.

3. Bioreaktor nach einem der vorangehenden Ansprüche,
**dadurch gekennzeichnet,**
**dass** der Profilring (16) eine umlaufende, zum Beutelinneren hin offene Führungsnut (162) aufweist, in der wenigstens ein radial außen an dem Rotor (18) angeordneter Führungsschuh (185) axial und radial geführt ist.

4. Bioreaktor nach Anspruch 3,
**dadurch gekennzeichnet,**
**dass** an wenigstens drei Umfangspositionen des Profilrings (16) radial ausgerichtete Wälzlagerrollen (163) und/oder axial ausgerichtete, gegenständige Paare von Wälzlagerrollen (164) federnd in die Führungsnut (162) hineinragen.

5. Bioreaktor nach Anspruch 3,
**dadurch gekennzeichnet,**
**dass** an wenigstens drei Umfangspositionen des Profilrings radial ausgerichtete Gleitlagerfedern und/oder axial ausgerichtete, gegenständige Paare von Gleitlagerfedern in die Führungsnut hineinragen.

6. Bioreaktor nach einem der Ansprüche 3 bis 5,
**dadurch gekennzeichnet,**
**dass** die Führungsnut (162) auf der Außenseite des Profilrings (16) einen Vorsprung mit wenigstens einer Axialanlagefläche (167) bildet, an der eine als separate Einheit oder Einheitengruppe ausgebildete elektrische Spulenanordnung (20) angelegt ist, mit der das magnetische Feld erzeugbar ist.

7. Bioreaktor nach Anspruch 6,
**dadurch gekennzeichnet,**
**dass** die Führungsnut (162) auf der Außenseite des Profilrings (16) dreiseitig von einer als separate Einheit oder Einheitengruppe ausgebildeten elektrischen Spulenanordnung (20), mit der das magnetisches Feld erzeugbar ist, formschlüssig umgriffen ist.

8. Bioreaktor nach einem der vorangehenden Ansprüche,
**dadurch gekennzeichnet,**
**dass** der Rotor (18) einen Zentralkörper (181) und wenigstens drei sich radial von dem Zentralkörper (181) erstreckende Speichen (184) aufweist.

9. Bioreaktor nach Anspruch 8,
**dadurch gekennzeichnet,**
**dass** die Speichen (184) des Rotors (18) durch eine ringförmige Felge (185) miteinander verbunden sind.

10. Bioreaktor nach Anspruch 9,
**dadurch gekennzeichnet,**
**dass** die Felge (185) mit einer Mehrzahl über ihren Umfang verteilter Permanentmagnete (186) versehen ist.

11. Reaktorbeutel für einen Bioreaktor (10),
umfassend eine überwiegend flexible Beutelwand (121), die zur Lagerung eines einer Umwälzung eines Beutelinhaltes dienenden, wellenlosen, wenigstens bereichsweise permanentmagnetischen Rührers (18) einen steif ausgebildeten Lagerbereich aufweist,
**dadurch gekennzeichnet,**
**dass** in einem Höhenabschnitt des Reaktorbeutels (12) als Lagerbereich ein steifer Profilring (16) aus einem unmagnetischen Werkstoff angeordnet ist, an dem ein als den Beutelquerschnitt überspannender Rotor (18) ausgebildeter Rührer mittels einer Schienenführung (162/185) drehbeweglich axial und radial lagerbar ist.

12. Reaktorbeutel nach Anspruch 11,
**dadurch gekennzeichnet,**
**dass** der Profilring (16) die flexible Beutelwand (121) durchsetzt und mit ihr stoffschlüssig verbunden ist.

13. Reaktorbeutel nach einem der Ansprüche 11 bis 12,
**dadurch gekennzeichnet,**
**dass** im Beutelinneren ein als ein wellenloser, den Beutelquerschnitt überspannender Rotor (18) ausgebildeter Rührer angeordnet und mittels einer Schienenführung (162/185) drehbeweglich an dem Profilring (16) axial und radial gelagert ist.

14. Rührer zur Umwälzung eines Inhaltes eines Bioreaktors (10), bereichsweise permanentmagnetisch ausgebildet und umfassend eine Mehrzahl flügelartiger, an einer Halterung fixierter Umwälzorgane(182),
**dadurch gekennzeichnet,**
**dass** der Rührer als ein einen Zentralkörper (181) und wenigstens drei sich von dem Zentralkörper(181) radial erstreckende Speichen (184) aufweisender Rotor ausgebildet ist, der in seinem radial äußeren Bereich mit wenigstens einem Permanentmagneten (186) versehen ist und wenigstens ein Führungsglied (185) einer äußeren Schienenführung (162/185) aufweist.

15. Rührer nach Anspruch 14,
**dadurch gekennzeichnet,**
**dass** die Speichen (184) des Rotors (18) durch eine ringförmige Felge (185), die mit einer Mehrzahl über ihren Umfang verteilter Permanentmagnete (186) versehen ist, miteinander verbunden sind.

## Claims

1. A bioreactor, comprising
- a reactor bag (12) having a predominantly flexible bag wall (121) which has a rigidly formed bearing area for housing a stirrer (18) serving to circulate the contents of the bag,
- a shaftless stirrer (18), parts of which are permanently magnetic, housed in the bearing area in the inside of the bag, and
- a coil arrangement (20) positioned outside the reactor bag (12), with which a rotating magnetic field can be produced that interacts, in a manner exerting a torque on the stirrer (18), with the permanent-magnetic areas thereof,
**characterized in that**
a rigid profile ring (16) made of a non-magnetic material, on which the stirrer formed as the rotor (18) spanning the cross-section of the bag is rotatably housed by means of a guide rail (162/185), is arranged as a bearing area in a segment of hight of the reactor bag (12),
the magnetic field of the electric coil arrangement (20) interacting with at least one permanent magnet (186) arranged in the radially outer area of the rotor (18).

2. The bioreactor according to Claim 1,
**characterized in that**
the profile ring (16) penetrates the flexible bag wall (121) and is bonded to it.

3. The bioreactor according to one of the preceding claims,
**characterized in that**
the profile ring (16) has a guide groove (162) running around it which is open towards the interior of the bag, into which guide groove at least one guide shoe (185) arranged on a radially outer part of the rotor (18) is introduced axially and radially.

4. The bioreactor according to Claim 3,
**characterized in that**
at no fewer than three positions on the circumference of the profile ring (16), radially oriented roller bearings (163) and/or axially oriented, opposed pairs of roller bearings (164) project with spring action into the guide groove (162).

5. The bioreactor according to Claim 3,
**characterized in that**
at no fewer than three positions on the circumference of the profile ring, radially oriented slide bearing springs and/or axially oriented, opposed pairs of slide bearing springs project into the guide groove.

6. The bioreactor according to any of Claims 3 to 5,
**characterized in that**
on the outer side of the profile ring (16), the guide groove (162) forms a projection with at least one axial mounting surface (167) on which an electric coil arrangement (20) designed as a separate unit or unit group is arranged, the coil arrangement (20) being capable of generating the magnetic field.

7. The bioreactor according to Claim 6,
**characterized in that**
the guide groove (162) on the outer side of the profile ring (16) is surrounded on three sides, by form-fitting connection, by an electric coil arrangement (20) designed as a separate unit or unit group, the coil arrangement (20) being capable of generating the magnetic field.

8. The bioreactor according to any of the preceding claims,
**characterized in that**
the rotor (18) has a central body (181) and at least three spokes (184) extending radially from the central body (181).

9. The bioreactor according to Claim 8,
**characterized in that**
the spokes (184) of the rotor (18) are connected to one another by a ring-shaped rim (185).

10. The bioreactor according to Claim 9,
**characterized in that**
the rim (185) has a plurality of permanent magnets (186) distributed around its circumference.

11. A reactor bag for a bioreactor (10),
comprising a predominantly flexible bag wall (121) which has a rigidly formed bearing area for housing a shaftless stirrer (18) serving to circulate the contents of the bag, at least parts of the stirrer being permanently magnetic,
**characterized in that**
a rigid profile ring (16) made of a non-magnetic material, on which a stirrer formed as a rotor (18) spanning the cross-section of the bag is housed rotatable in an axial and radial manner by means of a guide rail (162/185), is arranged as a bearing area in a segment of hight of the reactor bag (12).

12. The reactor bag according to Claim 11,
**characterized in that**
the profile ring (16) penetrates the flexible bag wall (121) and is bonded to it.

13. The reactor bag according to any of Claims 11 to 12,
**characterized in that**
a stirrer is arranged in the interior of the bag as a shaftless rotor (18) spanning the cross-section of the bag and is housed rotatable in an axial and radial manner on the profile ring (16) by means of a guide rail (162/185).

14. A stirrer to circulate the contents of a bioreactor (10), parts of which being permanently magnetic, and comprising a plurality of paddle-shaped circulation elements (182) fastened to a holder,
**characterized in that**,
the stirrer is designed as a rotor with a central body (181) having at least three spokes (184) extending radially from the central body (181), which rotor has at least one permanent magnet (186) arranged in its radially outer area and at least one guide element (185) of an outer guide rail (162/185).

15. The stirrer according to Claim 14,
**characterized in that**
the spokes (184) of the rotor (18) are connected to one another by a ring-shaped rim (185) that has a plurality of permanent magnets (186) distributed around its circumference.

## Revendications

1. Bioréacteur, comprenant
- une enveloppe de réacteur (12) dotée d'une paroi d'enveloppe (121) pour la plupart flexible, qui présente une région formant palier réalisée rigide pour le montage d'un agitateur (18) servant à faire circuler un contenu d'enveloppe,
- un agitateur (18) sans arbre, à aimantation permanente dans certaines régions, monté à l'intérieur de l'enveloppe sur la région formant palier,
- et un ensemble de bobines (20) positionné à l'extérieur de l'enveloppe de réacteur (12), ensemble qui permet de générer un champ magnétique rotatif entrant en interaction avec les régions à aimantation permanente de l'agitateur (18) d'une manière exerçant un couple de rotation sur ce dernier,
**caractérisé en ce qu'**une bague profilée rigide (16) réalisée en un matériau non magnétique est disposée comme région formant palier dans une partie de la hauteur de l'enveloppe de réacteur (12), bague sur laquelle l'agitateur réalisé sous la forme d'un rotor (18) enjambant la section de l'enveloppe est monté de manière mobile en rotation au moyen d'un guidage à rail (162/185),
sachant que le champ magnétique de l'ensemble de bobines électriques (20) entre en interaction avec au moins un aimant permanent (186) disposé dans la région radialement extérieure du rotor (18).

2. Bioréacteur selon la revendication 1,
**caractérisé en ce que** la bague profilée (16) traverse la paroi d'enveloppe flexible (121) et lui est assemblée par liaison de matière.

3. Bioréacteur selon l'une des revendications précédentes,
**caractérisé en ce que** la bague profilée (16) présente une rainure de guidage (162) s'étendant sur toute la circonférence et ouverte vers l'intérieur de l'enveloppe, rainure dans laquelle au moins un patin de guidage (185) disposé radialement à l'extérieur sur le rotor (18) est guidé axialement et radialement.

4. Bioréacteur selon la revendication 3,
**caractérisé en ce que**, en au moins trois points de la circonférence de la bague profilée (16), des rouleaux de roulement (163) orientés radialement et/ou des paires opposées de rouleaux de roulement (164) orientées axialement pénètrent avec effet de ressort dans la rainure de guidage (162).

5. Bioréacteur selon la revendication 3,
**caractérisé en ce que**, en au moins trois points de la circonférence de la bague profilée, des ressorts de palier lisse orientés radialement et/ou des paires opposées de ressorts de palier lisse orientées axialement pénètrent dans la rainure de guidage.

6. Bioréacteur selon l'une des revendications 3 à 5,
**caractérisé en ce que** la rainure de guidage (162) forme sur le côté extérieur de la bague profilée (16) une saillie dotée d'au moins une surface d'application axiale (167), contre laquelle est appliqué un ensemble de bobines électriques (20) réalisé sous forme d'unité séparée ou de groupe d'unités séparé, qui permet de générer le champ magnétique.

7. Bioréacteur selon la revendication 6,
**caractérisé en ce que** la rainure de guidage (162) sur le côté extérieur de la bague profilée (16) est entourée en engagement positif sur trois côtés par un ensemble de bobines électriques (20) réalisé sous forme d'unité séparée ou de groupe d'unités séparé, qui permet de générer le champ magnétique.

8. Bioréacteur selon l'une des revendications précédentes,
**caractérisé en ce que** le rotor (18) présente un corps central (181) et au moins trois rayons (184) s'étendant radialement à partir du corps central (181).

9. Bioréacteur selon la revendication 8,
**caractérisé en ce que** les rayons (184) du rotor (18) sont reliés entre eux par une jante annulaire (185).

10. Bioréacteur selon la revendication 9,
**caractérisé en ce que** la jante (185) est pourvue d'une pluralité d'aimants permanents (186) répartis sur sa circonférence.

11. Enveloppe de réacteur pour un bioréacteur (10), comprenant une paroi d'enveloppe (121) pour la plupart flexible, qui présente une région formant palier réalisée rigide pour le montage d'un agitateur (18) sans arbre, à aimantation permanente au moins dans certaines régions, servant à faire circuler un contenu d'enveloppe, **caractérisée en ce qu'**une bague profilée rigide (16) réalisée en un matériau non magnétique est disposée comme région formant palier dans une partie de la hauteur de l'enveloppe de réacteur (12), bague sur laquelle un agitateur réalisé sous la forme d'un rotor (18) enjambant la section de l'enveloppe peut être monté axialement et radialement en étant mobile en rotation au moyen d'un guidage à rail (162/185).

12. Enveloppe de réacteur selon la revendication 11,
**caractérisée en ce que** la bague profilée (16) traverse la paroi d'enveloppe flexible (121) et lui est assemblée par liaison de matière.

13. Enveloppe de réacteur selon l'une des revendications 11 à 12,
**caractérisée en ce qu'**un agitateur réalisé sous la forme d'un rotor (18) sans arbre enjambant la section de l'enveloppe est disposé à l'intérieur de l'enveloppe et est monté axialement et radialement sur la bague profilée (16) en étant mobile en rotation au moyen d'un guidage à rail (162/185).

14. Agitateur pour faire circuler le contenu d'un bioréacteur (10), réalisé à aimantation permanente dans certaines régions et comprenant une pluralité d'organes de circulation (182) du genre pales, fixés à un support, **caractérisé en ce que** l'agitateur est réalisé sous la forme d'un rotor présentant un corps central (181) et au moins trois rayons (184) s'étendant radialement à partir du corps central (181), rotor qui est pourvu d'au moins un aimant permanent (186) dans sa région radialement extérieure et qui présente au moins un organe de guidage (185) d'un guidage à rail (162/185) extérieur.

15. Agitateur selon la revendication 14,
**caractérisé en ce que** les rayons (184) du rotor (18) sont reliés entre eux par une jante annulaire (185) qui est pourvue d'une pluralité d'aimants permanents (186) répartis sur sa circonférence.
